# DEMANDE DE BREVET EUROPEEN

(11) **EP 3 879 267 A1**
(43) Date de publication de la demande: **15.09.2021**
(21) Numéro de dépôt: 21162120.6
(22) Date de dépôt: 11.03.2021
(51) Int. Cl.: G01N 33/18

(54) **PROCÉDÉ DE MESURE EN LIGNE DU BESOIN BIOLOGIQUE D'UN BASSIN DANS UNE STATION DE TRAITEMENT DES EAUX**

(30) Priorité: 12.03.2020 FR 2002460
(71) Demandeur: L'AIR LIQUIDE, SOCIETE ANONYME POUR L'ETUDE ET L'EXPLOITATION DES PROCEDES GEORGES CLAUDE, 75007 Paris (FR); Air Liquide France Industrie, 75007 Paris (FR)
(72) Inventeur: ALBAN, Bruno, 92227 Bagneux (FR); BEAUDOUIN, Guillaume, 92227 Bagneux (FR); CAMPO, Philippe, 92227 Bagneux (FR)
(74) Mandataire: Air Liquide

(57) **Abrégé**

Un procédé de détermination en ligne du besoin en oxygène d'un bassin (10) biologique à boues activées, ou d'une zone d'un bassin biologique à boues activées, dans une station de traitement des eaux, où l'on met en œuvre les étapes suivantes :
- on effectue un prélèvement (12, V1, V2, V3, V4) d'un échantillon de liqueur mixte, prélèvement effectué dans le bassin ou dans une zone du bassin où l'on souhaite déterminer le besoin en oxygène ;
- on introduit le prélèvement dans un récipient (6), récipient préférentiellement équipé d'un système d'agitation et d'un système d'injection (4) d'air ou d'un gaz riche en oxygène ;
- on mesure la teneur en oxygène dissous initiale de l'échantillon et on met en route le cas échéant l'agitation ;
- selon la position de la teneur en oxygène dissous initiale de l'échantillon on ordonne ou non l'injection d'un gaz contenant de l'oxygène dans l'échantillon, et l'on réalise un enregistrement de l'évolution de la teneur en oxygène dissous et ainsi de la consommation en oxygène du milieu présent dans le récipient au cours du temps.

## Description

La présente invention concerne le domaine du traitement des eaux usagées.

Une station de traitement des eaux usées peut s'avérer délicate à contrôler. De nombreux travaux et équipements ont été réalisés et développés dans le passé de façon à améliorer la compréhension quotidienne de la marche d'une telle station, ce qui permet de faire de meilleurs choix quant à l'évolution des très nombreux paramètres opératoires intervenant dans la marche de telles installations.

Ce bilan quotidien comprenant un suivi des actions permettant l'évolution et l'optimisation de la station est dévolu à des personnes du site, il représente un travail significatif et quotidien (coût opératoire, énergie consommée, aération/injection d'air ou d'oxygène en particulier).

On rappellera qu'une station est équipée traditionnellement d'un ensemble de moyens de mesure :
- Certaines mesures que l'on peut qualifier de « simples » : il s'agit d'instruments utiles au contrôle des procédés de façon générale (de traitement des eaux ou non) tels que débitmètres, sondes de température, de pH, d'oxygène dissous, manomètres... mesures qui permettent d'avoir des valeurs instantanées.
- En complément, les installations sont équipées de moyens de mesures plus spécifiques aux stations d'eaux usées, qui donnent accès à des informations « globales » telles que le COT (« Carbone Organique Total »), la DCO (Demande Chimique en Oxygène) et autres.

Mais force est de constater que cette situation existante n'est pas pleinement satisfaisante :
- D'une part, ces mesures directes ou indirectes en ligne donnent accès à des résultats qui sont parfois contestables du fait de la variabilité de la qualité de l'effluent entrant.
- D'autre part, ces mesures, le plus souvent, ne sont pas utilisées directement, les résultats servent en premier lieu à déterminer la charge polluante qui entre sur la station et ainsi déterminer les paramètres de réglages nécessaires pour traiter cette charge polluante, en particulier la quantité d'air ou d'oxygène à injecter pour traiter efficacement sur un bassin aérobie la pollution entrante. Les réglages sont donc principalement faits à postériori lorsque la charge polluante est passée.

La présente invention s'attache alors à proposer une solution technique pour améliorer cette situation non satisfaisante.

Comme on le verra plus en détails dans ce qui suit, la présente invention propose d'installer un équipement simple, capable, de façon séquentielle, plusieurs fois par jour et en quelques minutes, de déterminer le besoin en oxygène REEL d'un bassin biologique, nécessaire pour la respiration endogène et exogène nécessaire pour dégrader la charge polluante.

A partir de cette mesure rapide, il est possible d'ajuster directement la marche de la station, ce qui permettra de mieux maîtriser et d'anticiper les actions à mettre en œuvre pour répondre à une dérive du besoin notamment à une dérive des caractéristiques de l'effluent entrant.

A titre illustratif :
- En comparant l'évolution du besoin tel qu'évalué selon la présente invention à différents moments successifs, on pourra repérer une déviation : par exemple un besoin en oxygène faible alors que le débit d'effluent en entrée de station est stable et une concentration en oxygène dissous conforme aux attentes, cela pourra indiquer une baisse de la pollution contenue dans l'effluent à traiter. Toujours à titre d'exemple, dans le cas de dysfonctionnements provoqués par un ou plusieurs composés toxiques pour les bactéries (par exemple du fait de l'accumulation de CO₂ dissous).
   Ainsi toujours à titre d'exemple :
   - on mesure à l'instant t1 un besoin en oxygène de 100 kg/h pour un débit d'effluent de 200 m³/h et une concentration en oxygène dissous de 0,5 mg/l
   - on mesure à l'instant t2 un besoin en oxygène de 30 kg/h pour le même débit d'effluent et une concentration en oxygène dissous constante de 0,5 mg/l
   - on va alors pouvoir alerter sur deux cas de figure possibles :
      a) soit la concentration en polluant dans les 200 m3/h d'effluent baisse, mais cela le site producteur doit en avoir conscience il sait ce qu'il produit.
      b) soit il y a un dysfonctionnement biologique qui est en train de se produire, les micro-organismes réduisent leur activité (toxique ou autre) et consomment moins.
- En comparant le besoin évalué selon l'invention à la totalité de l'oxygène injecté dans la station (provenant d'air ou d'oxygène pur), le rapport conduira à l'estimation du rendement des appareils d'aération, ce qui permettra de détecter des défauts, des casses, ou encore le vieillissement des systèmes d'injection par exemple.

La présente invention peut être mise en œuvre facilement sur un bassin biologique à boues activées considéré comme parfaitement mélangé. Dans le cas de bassins de type flux piston (chenal ou autres, l'homme du métier est familier de ces différents types de bassins), le besoin ne peut être déterminé que par zone, dans ce cas il sera préférable :
- soit de dupliquer l'équipement de l'invention,
- soit préférentiellement d'envoyer dans l'équipement de l'invention de la liqueur mixte (c'est-à-dire de l'effluent avec des boues activées) prélevée dans la zone sur laquelle on souhaite faire la mesure

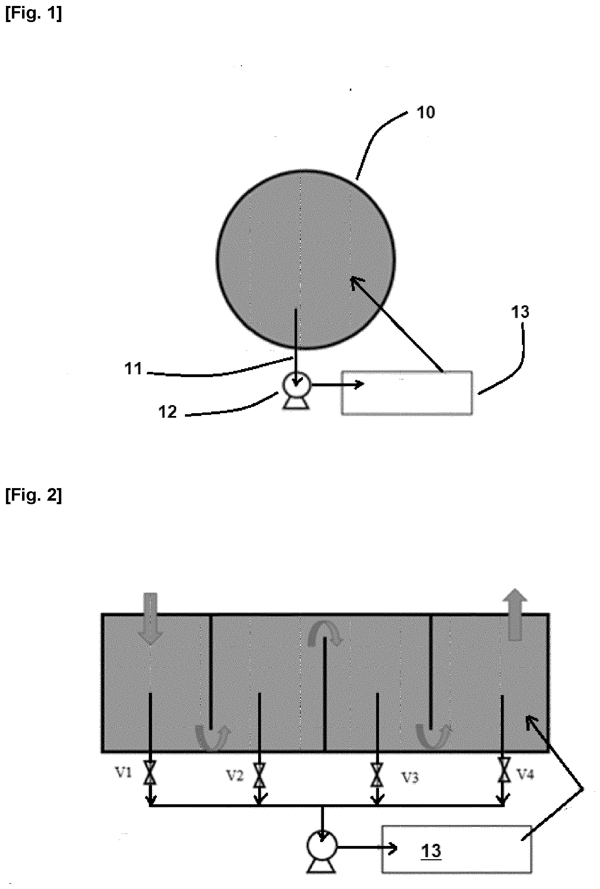

Considérons dans ce qui suit un exemple de mise en œuvre sur un bassin parfaitement mélangé à l'aide de la figure 1 annexée.

On prélève du bassin (10) un échantillon (11) de quelques litres à l'aide de la pompe 12, typiquement 2 à 10 litres, que l'on envoie dans l'équipement (13) de détermination du besoin en oxygène conforme à l'invention, échantillon renvoyé dans le bassin après mesure.

On illustre grâce à la figure 2 annexée un exemple de mise en œuvre permettant, dans le cas d'un bassin non homogène (type à flux piston), à l'aide d'un seul équipement, par le prélèvement et l'envoi successif de la boue activée vers l'équipement (mode séquentiel), de mesurer le besoin en oxygène dans différentes zones d'un bassin de traitement biologique.

On prélève un échantillon de quelques litres, typiquement 2 à 10 litres, d'une zone donnée du bassin, par l'ouverture d'une des électrovannes V1, V2, V3, V4 , que l'on envoie dans l'équipement 13 de détermination du besoin en oxygène conforme à l'invention, échantillon renvoyé dans le bassin après mesure.

L'équipement de détermination du besoin en oxygène selon l'invention met en œuvre le mode d'opération suivant : un échantillon de liqueur mixte, c'est-à-dire de l'effluent avec des boues activées, est prélevé dans la zone du bassin où l'on souhaite déterminer le besoin en oxygène. Comme décrit précédemment, l'échantillon peut être prélevé n'importe où sur un bassin parfaitement mélangé ou dans une zone particulière sur un bassin non homogène. Ce prélèvement pourra être réalisé par une pompe ou par écoulement gravitaire.

Le prélèvement, typiquement de l'ordre de 2 à 10 litres, est introduit dans un récipient équipé préférentiellement d'un système d'agitation (agitateur, pompe ou autres système), et d'un système d'injection d'air ou préférentiellement d'un gaz riche en oxygène (oxygène pur par exemple).

On décrit ici l'injection d'air ou d'un gaz riche en oxygène, mais il faut noter que dans certaines situations, cette injection se révélera inutile, tout simplement car dans certaines situations (certaines origines d'échantillons), l'échantillon est « déjà » riche en oxygène dissous quand il parvient dans l'appareil, dans ce cas il n'y a pas nécessité d'enrichir plus l'échantillon et on prend alors directement la courbe de consommation comme on va le décrire ci-dessous.

En pratique on peut considérer que si l'échantillon comporte initialement moins de 4 mgO₂/l, on va injecter un gaz d'enrichissement (air, oxygène pur, Air + O₂).

Juste après l'introduction de l'échantillon dans le récipient, l'agitation est mise en service avec une injection du gaz contenant de l'oxygène, ceci afin de "monter " rapidement la concentration en oxygène dissous dans l'échantillon. L'utilisation d'un gaz riche en oxygène permet de limiter la durée de montée, ce qui évite par la suite un temps de mesure trop long. En effet, ce temps trop long impacterait l'exactitude du résultat du fait de la consommation d'une partie de la pollution pendant ce temps.

On peut noter qu'il n'est pas nécessaire d'atteindre la saturation dans l'échantillon. Quelques mg/l d'oxygène suffisent (par exemple de l'ordre de 3 à 20 mg/l).

Lorsque la valeur cible (entre 3 et 40 mg/l, préférentiellement entre 4 et 15 mg/l) est atteinte, l'injection du gaz riche en oxygène est arrêtée alors que l'agitation est conservée. On réalise alors un enregistrement de la consommation de l'oxygène au cours du temps du milieu présent dans le récipient.

Une sonde de mesure d'oxygène placée dans le récipient permet d'effectuer cette détermination et cet enregistrement.

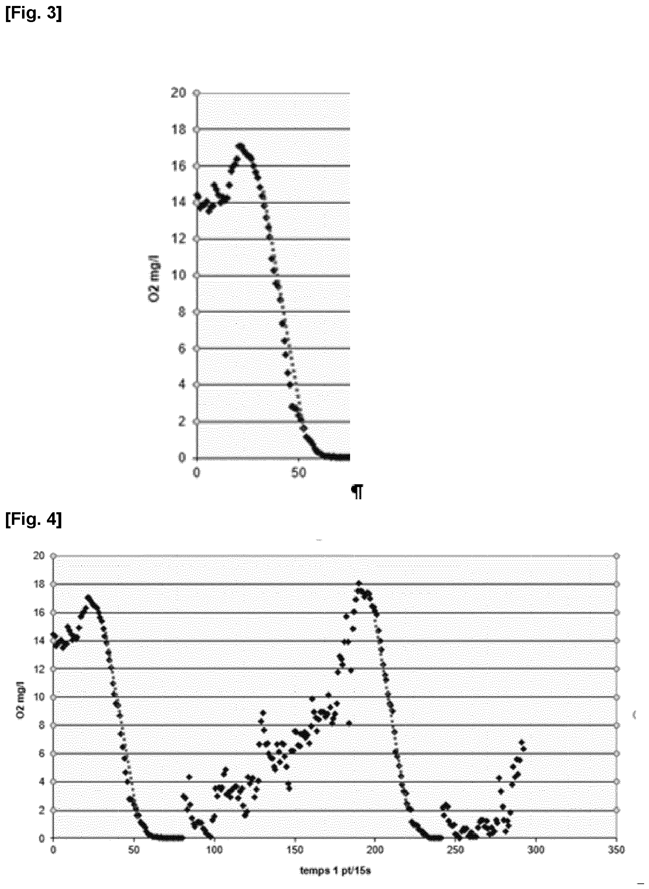

Pour exemple, l'enregistrement de l'oxygène permet d'obtenir le type de courbe exemplifié en figure 3 annexée. En ordonné, la concentration en oxygène en mg/l et en abscisse, le temps pendant lequel la mesure est réalisée (en secondes).

Et l'on observe sur la courbe les phénomènes suivants :
- le début de la courbe correspond à la fin de la période d'injection du gaz riche en O₂ (dans notre cas de l'oxygène pur),
- et la deuxième partie de la courbe (descente) correspond à la pente de consommation d'oxygène de l'échantillon.

Il est bien évidemment possible de répéter la mesure à plusieurs reprises pour avoir une répétabilité de la mesure. Il est néanmoins hautement préférable de renouveler à chaque fois la mesure avec un échantillon fraichement prélevé.

A titre d'exemple de répétabilité de la mesure on peut se reporter à la figure 4 annexée, où l'on observe les résultats d'oxygène obtenus lorsque la mesure est réalisée deux ou plusieurs fois, avec à chaque fois un échantillon fraichement prélevé.

Comme indiqué précédemment, la pente de la droite de descente correspond à la chute de concentration en oxygène dissous dans l'échantillon pendant la période d'observation. Elle correspond donc au besoin biologique en oxygène pour dégrader la pollution biodégradable contenu dans l'échantillon et pour la respiration endogène des microorganismes.

Ainsi dans ce graphe on constate que la différence d'oxygène dissous observée sur 6 minutes sur les droites est de 12,7 mg/l, différence qui correspond donc à la consommation de l'échantillon pendant la mesure.

On peut alors ramener cette valeur dans l'unité que l'on souhaite (en consommation par heure, soit ici 127 mg d'oxygène par litre d'échantillon et par heure par exemple).

Et dans le cas d'un bassin parfaitement mélangé, par volume du bassin, ou de la zone sur laquelle on réalise la mesure pour un bassin non parfaitement mélangé.

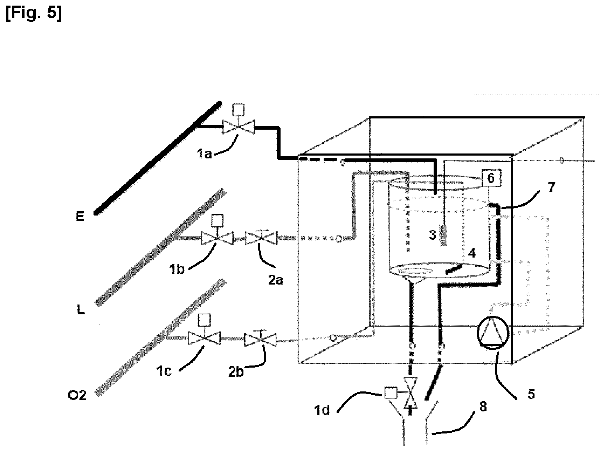

La figure 5 annexée permet de visualiser un exemple d'équipement conforme à l'invention, permettant de réaliser cette mesure en ligne du besoin biologique en oxygène sur un bassin.

On retrouve sur la figure 5 les éléments suivants :
- les références 1i : des électrovannes
- les références 2i : des vannes simples
- 3 : une sonde de mesure d'oxygène dissous
- 4 : un injecteur d'un gaz riche en oxygène
- 5 : une pompe pour générer une circulation de l'échantillon
- 6 : un récipient pour réaliser les mesures.
- 7 : surverse (trop plein) du contenant.
- 8 : égout.
   - L'alimentation en eau claire (E) permet le rinçage du système entre chaque mesure.
   - L'alimentation en liqueur mixte (L) correspond au prélèvement sur lequel on réalise la détermination du besoin en oxygène.
   - L'alimentation en oxygène (O₂) permet la montée rapide de la concentration en oxygène de l'échantillon.

L'invention concerne alors un procédé de mesure en ligne du besoin en oxygène d'un bassin biologique à boues activées, ou d'une zone d'un bassin biologique à boues activées, dans une station de traitement des eaux, où l'on met en œuvre les étapes suivantes :
- on effectue un prélèvement d'un échantillon de liqueur mixte, c'est-à-dire de l'effluent à traiter dans la station mélangée aux boues activées, prélèvement effectué dans le bassin ou dans une zone du bassin où l'on souhaite déterminer le besoin en oxygène ;
- on introduit le prélèvement dans un récipient, récipient préférentiellement équipé d'un système d'agitation et d'un système d'injection d'air ou d'un gaz riche en oxygène (de l'oxygène pur par exemple), ainsi que d'une sonde de mesure de la teneur en oxygène dissous du milieu présent dans le récipient ;

- après l'introduction de l'échantillon dans le récipient, on mesure la teneur en oxygène dissous initiale de l'échantillon et on met en route le cas échéant l'agitation ;
- si la teneur en oxygène dissous initiale de l'échantillon est supérieure ou égale à une consigne donnée, on observe la décroissance de la teneur en oxygène dissous dans l'échantillon, mesurée par la sonde, et ainsi la consommation en oxygène du milieu présent dans le récipient au cours du temps ;
- si la teneur en oxygène dissous initiale de l'échantillon est inférieure à ladite consigne donnée, on ordonne l'injection du gaz contenant de l'oxygène dans l'échantillon, et l'on observe l'augmentation de la teneur en oxygène dissous mesurée par la sonde, et lorsque qu'une valeur cible d'oxygène dissous est atteinte dans l'échantillon, on arrête l'injection du gaz riche en oxygène et l'on réalise un enregistrement de l'évolution de l'oxygène dissous mesuré par la sonde et ainsi de la consommation en oxygène du milieu présent dans le récipient au cours du temps.

Comme il apparaitra clairement à l'homme du métier à la lecture de ce qui précède, c'est donc le grand mérite et le caractère remarquable de la présente invention de proposer une configuration où l'on met en œuvre un récipient agité ou réacteur, qui est placé en dehors du bassin biologique dont on veut mesurer la demande ou besoin biologique. L'échantillon à évaluer ou échantillon de liqueur mixte est donc introduit puis évacué après la mesure.

Le principe de mesure dit « respirométrique », habituellement réalisé à la main sur un échantillon prélevé, est ici automatisé.

Le fait de disposer d'un équipement à part, séparé du bassin, présente de nombreux avantages, avantages cruciaux :
- le matériel au centre du dispositif est simplifié au maximum : un seul réacteur ou volume, un seul moyen d'agitation et une seule sonde de mesure d'oxygène dissous, d'où une économie sur le coût de mise en œuvre de la solution et une maintenance simplifiée.
- on dispose de toute la souplesse pour réaliser la respirométrie dans les meilleures conditions. La possibilité de pré-aérer l'échantillon prélevé ou pas, en fonction de sa teneur initiale en oxygène dissous par exemple.
- les soucis d'opération sont limités au maximum. L'équipement est choisi et dimensionné de telle sorte qu'on limite au maximum l'encrassement ou les dépôts (forme du récipient agité par exemple).
- ce système, couplé à plusieurs petites pompes disposées à différents endroits dans un bassin, permettent de mesurer le besoin à différents endroits : l'un après l'autre par exemple, plusieurs à la suite sur le même prélèvement si évolution constatée. Le matériel est très limité (pompes et tuyauterie principalement) mais il permet de suivre au mieux un bassin biologique. C'est particulièrement vrai pour les bassins de forme chenaux. Ceci n'est pas possible lorsque la mesure est réalisée à même le bassin.

## Revendications

1. Procédé de mesure en ligne du besoin en oxygène d'un bassin (10) biologique à boues activées, ou d'une zone d'un bassin biologique à boues activées, dans une station de traitement des eaux, où l'on met en œuvre les étapes suivantes :
- on effectue un prélèvement (12, V1, V2, V3, V4) d'un échantillon de liqueur mixte, c'est-à-dire de l'effluent à traiter dans la station mélangé aux boues activées, prélèvement effectué dans le bassin ou dans une zone du bassin où l'on souhaite déterminer le besoin en oxygène ;
- on introduit le prélèvement dans un récipient (6), récipient préférentiellement équipé d'un système d'agitation et d'un système d'injection (4) d'air ou d'un gaz riche en oxygène, ainsi que d'une sonde (3) de mesure de la teneur en oxygène dissous du milieu présent dans le récipient ;
- après l'introduction de l'échantillon dans le récipient, on mesure la teneur en oxygène dissous initiale de l'échantillon et on met en route le cas échéant l'agitation ;
- si la teneur en oxygène dissous initiale de l'échantillon est supérieure ou égale à une consigne donnée, on observe la décroissance de la teneur en oxygène dissous dans l'échantillon, mesurée par la sonde, et ainsi la consommation en oxygène du milieu présent dans le récipient au cours du temps ;
- si la teneur en oxygène dissous initiale de l'échantillon est inférieure à ladite consigne donnée, on ordonne l'injection du gaz contenant de l'oxygène dans l'échantillon, et l'on observe l'augmentation de la teneur en oxygène dissous mesurée par la sonde, et lorsque qu'une valeur cible d'oxygène dissous est atteinte dans l'échantillon, on arrête l'injection du gaz riche en oxygène et l'on réalise un enregistrement de l'évolution de l'oxygène dissous mesuré par la sonde et ainsi de la consommation en oxygène du milieu présent dans le récipient au cours du temps.
